(19) **Europäisches Patentamt European Patent Office Office européen des brevets**

(11) **EP 1 769 739 B1**

(12) **EUROPÄISCHE PATENTSCHRIFT**

(45) Veröffentlichungstag und Bekanntmachung des Hinweises auf die Patenterteilung:
**27.05.2015 Patentblatt 2015/22**

(51) Int Cl.:
***A61B 5/0225*** *(2006.01)*

(21) Anmeldenummer: **06020564.8**

(22) Anmeldetag: **29.09.2006**

(54) **Vorrichtung und Verfahren zur Blutdruckmessung**

Blood pressure measurement device and method

Appareil et procédé de mesure de la pression sanguine

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **29.09.2005 EP 05021330**

(43) Veröffentlichungstag der Anmeldung:
**04.04.2007 Patentblatt 2007/14**

(60) Teilanmeldung:
**10186038.5 / 2 289 406**

(73) Patentinhaber: **Egner, Wolfgang**
**63533 Mainhausen (DE)**

(72) Erfinder:
• **Egner, Wolfgang**
**63533 Mainhausen (DE)**
• **Egner, Beate**
**63533 Mainhausen (DE)**

(74) Vertreter: **Stoffregen, Hans-Herbert**
**Patentanwalt**
**Postfach 21 44**
**63411 Hanau (DE)**

(56) Entgegenhaltungen:
**EP-A- 1 057 450    EP-A2- 0 651 969**
**DE-A- 4 230 693    DE-A- 4 300 966**
**DE-A1- 19 700 115    US-A- 6 068 601**

Printed by Jouve, 75001 PARIS (FR)

**Beschreibung**

Die vorliegende Erfindung bezieht sich auf eine Vorrichtung nach dem Oberbegriff des Anspruchs 1 sowie auf ein Verfahren nach dem Oberbegriff des Anspruchs 11.

**Hintergrund der Erfindung**

Eine Vorrichtung und ein Verfahren zur Blutdruckmessung der eingangs genannten Art ist in der DE-A-43 00 966 offenbart. Diese bezieht sich auf eine Signalverarbeitungseinheit, insbesondere zur automatischen Blutdruckmessung oder Patientenüberwachung, und ein zugehöriges Blutdruckmess- bzw. Patientenüberwachungsgerät. Eine Manschette liefert ein Drucksignal, das Blutdruckpulse enthält. Ein Mikroprozessor filtert das Drucksignal mittels Filter und erzeugt ein Hilfssignal mit Spitzen an den Anfangspunkten der Pulse zur Bildung eines weiteren Drucksignals. Nach Abzug einer Komponente vom Drucksignal ergibt sich das reine Blutdrucksignal. Der Mikroprozessor berechnet über eine Funktion den mittleren, systolischen und diastolischen Blutdruckwert.

Die DE-A-42 30 693 bezieht sich auf ein automatisches Blutdruckmessgerät mit einem flexiblen Aufpumpsystem zum schnellen und exakten Aufpumpen von Neonatal-, pädiatrischen und Erwachsenenmanschetten ohne signifikante Drucküberschreitung. Das Blutdruckmessgerät umfasst eine elektrische Pumpe und zwei Eintrittsblenden. Eine eine reduzierte Strömung bewirkende Blende versorgt die Pumpe mit Luft für alle Manschettengrößen. Eine Blende ohne Reduzierung, die durch Betätigung eines Ventils geöffnet oder geschlossen werden kann, versorgt die Pumpe nur dann mit Luft, wenn eine Erwachsenen- oder eine pädiatrische Manschette benutzt wird. Außerdem wird die Pumpe über einen Motorregler gesteuert, der einen dynamischen Ausschalteffekt bewirkt, um die Pumpe schnell zu stoppen, wenn der Druckgrenzwert erreicht ist. Ein Überdruckmechanismus, der getrennt von dem die Pumpe steuernden Drucksensor arbeitet, entzieht der Pumpe Betriebsstrom, wenn ein vorbestimmter Manschettendruckwert, der größer als der Druckgrenzwert ist, erreicht ist.

Ein weiteres Verfahren und eine weitere Vorrichtung zur Blutdruckmessung ist in der US-B-6,068,601 beschrieben. Die Vorrichtung umfasst eine aufblasbare Manschette, welche um einen Körperabschnitt eines lebenden Subjekts gebunden werden kann, eine Luftpumpe, welche Druckluft in die Manschette leitet und somit den Luftdruck in der Manschette erhöht, so dass die Manschette den Körperabschnitt des Subjekts mit Druck beaufschlagt, einen Lufttank, welcher mit der Luftpumpe verbunden ist, eine Drossel, welche zwischen dem Lufttank und der Manschette angeordnet ist, ein Entlüftungssteuerungsventil, welches zwischen der Drossel und der Manschette zur Steuerung einer Auslassmenge der Druckluft aus der Manschette vorgesehen ist und dadurch die Entlüftung der Manschette steuert. Ferner sind Steuergeräte zur Steuerung der Luftpumpe vorgesehen, welche die Manschette mit Druckluft versorgt, während das Entlüftungssteuerungsventil eine kontrollierte Menge von Druckluft aus der Manschette entlässt, um so den Druck der Manschette linear bis zu einer vorbestimmten ersten Rate zu erhöhen.

In der EP-A-0 651 969 wird ein Blutdrucküberwachungssystem beschrieben, welches einen Drucksensor mit einer Druckfläche und druckempfindlichen Elementen in der Druckfläche umfasst. Ferner ist ein Druckgerät vorgesehen, welches den Drucksensor gegen ein Arteriengefäß über eine Körperoberfläche presst, so dass jedes druckempfindliche Element einen Druckwert an der Körperoberfläche misst. Ferner sind zur Bestimmung einer optimalen Druckkraft für das Druckgerät vorgesehen, bei welchem ein Teil einer Wand des Arteriengefäßes unter dem Druck des Sensors abgeflacht ist. Ferner sind Mittel zur Variierung der Andruckkraft des Druckgerätes vorgesehen und zur Bestimmung eines Wendepunktes einer Kurve, welche die Beziehung zwischen der Änderung der Anpresskraft und durch den Drucksensor an der Körperfläche gemessenen Druckwerte repräsentiert. Schließlich sind Mittel zur Bestimmung eines Korrekturwertes basierend auf dem Druckwert eines bestimmten Wendepunktes und Blutdruckbestimmungsmittel zum Betrieb des Druckgerätes zur Aufrechterhaltung einer bestimmten optimalen Presskraft und Anpressen des Drucksensors gegen das Arteriengefäß über die Körperoberfläche und kontinuierliche Bestimmung von intra-arteriellen Blutdruckwerten der Arterie durch Subtraktion des Korrekturwertes von dem Druckwert, welcher durch den Drucksensor an der Körperoberfläche gemessen wurde.

In der DE-A-197 00 115 sowie der EP-A-1 057 450 ist eine Vorrichtung zum Ermitteln einer Übungsfunktion einer Person, die einer Übungsbelastung ausgesetzt ist, beschrieben. Die Vorrichtung umfasst eine Pulszahlmessvorrichtung, die eine Pulszahl der Person synchron mit dem Herzschlag der synchron aufeinanderfolgend misst, eine Blutdruckmessvorrichtung, die einen Blutdruck der Person synchron mit dem Herzschlag der Person aufeinanderfolgend und nicht invasiv misst, eine erste Berechnungsvorrichtung, die ein Produkt der Pulszahl und des Blutdrucks synchron mit dem Herzschlag der Person aufeinanderfolgend berechnet, eine zweite Berechnungsvorrichtung, die eine Gesamtenergie berechnet, die durch die Person erzeugt wird, nachdem mit dem Aufbringen der Übungsbelastung auf die Person begonnen wurde und bevor die Produkte, die durch die erste Berechnungsvorrichtung aufeinanderfolgend berechnet werden, auf einen Sollwert erhöht wurden, und eine Anzeigevorrichtung, die einen Ermittlungswert der Übungsfunktion der Person auf der Grundlage der Gesamtenergie, die durch die zweite Berechnungsvorrichtung berechnet wurde, anzeigt.

Die beschriebene Blutdruckmessvorrichtung umfasst eine aufblasbare Manschette, die über ein Leitungssystem mit einem Drucksensor, einem Wählventil und einer ersten Luftpumpe verbunden ist. Das Wählventil wird unter der Steuerung einer elektronischen Steuervorrichtung auswählend in einen ersten Zustand, in dem das Ventil startet, so dass Druckluft von der Luftpumpe zur Manschette geführt wird, um den Druck der Manschette zu erhöhen, einen zweiten Zustand, in dem das Ventil bewirkt, so dass sich die Manschette langsam entleert, und einen dritten Zustand, in dem das Ventil bewirkt, dass sich die Manschette schnell entleert, gebracht.

Ergänzend ist noch ein Druckpulswellensensor vorgesehen, d. h. ein behältnisartiges Sensorgehäuse und ein Paar von Befestigungsbändern, die mit dem Sensorgehäuse verbunden sind. Mit Hilfe der Befestigungsbänder wird der Druckpulswellen-Sensor an einer Faust des Arms des Patienten, an dem die Manschette getragen wird, oder am anderen Arm des Patienten lösbar befestigt, so dass eine Öffnung des Sensorgehäuses einer Körperfläche oder der Haut des Patienten gegenüberliegt. Ein Presselement ist über eine elastische Membran an Innenflächen des Sensorgehäuses befestigt, so dass das Presselement bezüglich des Gehäuses bewegbar ist durch die Öffnung des Gehäuses zur Körperoberfläche des Patienten hin vorbewegbar ist. Das Presselement weist einen Halbleiterchip auf, das aus monikristallinem Silizium ausgebildet ist, mit der flachen Fläche und eine Anzahl von Druckmesshalbleiterelementen, die in der flachen Oberfläche in einem Feld mit regelmäßigen Abstandsintervallen angeordnet sind.

Die derzeit auf dem medizinischen Markt der Blutdruckmessung üblichen Messsysteme arbeiten nicht im gesamten relevanten Druckbereich linear und es kommt daher in den oberen und unteren Grenzbereichen zu Abweichungen bzw. Messfehlern. Dies bestätigen auch R. L. Stepien et al. ("Comparative diagnostic test characteristics of oscillometric and doppler ultrasonographic methods in the detection of systolic hypertension in dogs", J. Vet. Intern. Med., 17:65-72, 2003).

Je nach Manschettenvolumen im Verhältnis zum Durchmesser des zur Messung verwendeten Probandenglieds wird meist nur in bestimmten Druckbereichen linear gemessen, üblicher Weise im Bereich zwischen 160 und 60 mm Hg (siehe Stepien et al., *supra*). Sowohl Menschen wie auch Tiere haben aber durchaus erheblich höhere oder niedrigere Werte, deren Messung wichtige diagnostische Information bereitstellen kann. Drücke, die mit herkömmlichen Blutdruckmessgeräten unter oder über diesem linearen Bereich gemessen werden, weichen meist massiv vom tatsächlichen Wert *in vivo* ab. Bei solchen Patienten, die einen Blutdruck außerhalb des Normbereichs des Gerätes aufweisen, wird daher oft falsch gemessen und folglich auch falsch diagnostiziert.

Des Weiteren verfälschen Manschetten, die zu fest oder zu locker angelegt werden, den tatsächlichen Druck, da die Arterie in diesem Fall entweder zu lange geschlossen ist, was zu einer zu niedrigen Anzeige des arteriellen systolischen Drucks führt, oder aber der Druck auf die Arterie ist so gering, dass die Stauungsamplituden sich ähnlich denen des wiedereintretenden Blutflusses darstellen, wodurch der systolische Druck als zu hoch anzeigt wird.

Viele Blutdruckmessgeräte verarbeiten nur eine Druckablassrate von 3 mm Hg pro Sekunde. Bei Menschen wird davon ausgegangen, dass der durchschnittliche Puls bei 60 x / Min. liegt. Man erhält also jede Sekunde etwa einen Pulsschlag. Daraus folgt, dass bei Übergehen eines Pulsschlages maximal nur eine Abweichung von 5 mm Hg des tatsächlichen Drucks auftreten kann. Dieser Fehler von 5 mm Hg wird von der Blutdruckliga in Deutschland als Standardfehlerwert akzeptiert. Bei einem Puls von mehr oder weniger als 60 / Min. kann der Fehler aber erheblich höher sein. Die derzeit üblichen osziflometrischen Biutdruckmessgeräte berücksichtigen aber den Einfluss des Pulses auf die Blutdruckmessung und damit den Einfluss auf die Ablassrate nur in einem sehr eingeschränkten Bereich bis ca. 160 Schläge/Minute,

Zudem richten sich alle Geräte nicht nach der maximalen Höhe der einzelnen Amplitude. Die einzelne Amplitude ist jedoch von der Größe des Herzens bzw, dem Leistungszustand (z.B. Leistungssportler vs. herzkranker Patient) des Herzens abhängig. Stattdessen wird herkömmlicher Weise eine maximale und eine minimale Amplitude für alle Menschen definiert und diese ergeben ein so genanntes Standardmesafenster. Das bedeutet, dass bei einer Amplitude mit Maximalwert oberhalb des definierten Fensters eine korrekte Berechnung nicht möglich ist, da der tatsächliche Wert nicht in die Berechnung eingeht Das Gleiche gilt für sehr niedrige Drücke. Man erkennt in diesen Fällen oft nicht genau den Zeitpunkt, zu dem die Arterie öffnet.

Zudem führen Artefakte wie Zittern. Bewegungen und Muskelzucken oft zu ähnlichen Amplituden wie der Blutdruck. Wenn man also nicht genau die Höhe, Breite und Länge einer Amplitude kennt, kann man nicht genau sagen, ob es ein Artefakt oder tatsächlich eine Blutdruckamplitude ist. Bei Systemen, die sich nur an der Größe orientieren, wird ein solches Artefakt durchaus fälschlich als realistischer Amplitudenwert angesehen.

Derzeit übliche Blutdruckmessgeräte bestehen aus einer aufblasbaren Manschette (Druckaufnehmer) und einem Ablassventil zum Verringern des Überdrucks sowie einer numerischen Anzeige des Drucks, ggf. unter Verwendung eines Wandlers, der analoge Oszillationen in digitale Druckwerte umwandelt.

Die in herkömmlichen Blutdruckmeßgeräten verwendeten mechanischen Ventile sind nicht in der Lage, einen zeitlich konstanten, d. h. linearen Druckabfall über den gesamten Druckbereich bereitzustellen. Auch sind bislang elektronische Ventile in Blutdruckmeßgeräten nie derartig gesteuert oder geregelt worden, dass sie einen zeitlich konstanten Druckabfall über den gesamten Druckbereich bereitstellen. Bis jetzt gibt es folglich kein Blutdruckmesssystem, das eine lineare Arbeitsweise über den gesamten Druckbereich ermöglicht.

**BESCHREIBUNG DER ERFINDUNG**

Es ist eine Aufgabe der vorliegenden Erfindung, eine Vorrichtung bzw ein Verfahren zur Blutdruckmessung bereitzustellen, das genaue Blutdruckmesswerte für Säugetiere aller Art im Bereich von etwa 5 bis über 300 mm Hg, z.B. bis 450 mm Hg bereitstellt. Zudem soll der Gegenstand der Erfindung Artefakte von Bfutdruckampiituden unterscheiden können und/oder unabhängig vom tatsächlichen Puls und Leistungszustarid bzw. Volumen des Hebens des untersuchten Patienten einen möglichst fehlerfreien tatsächlichen Blutdruck bereitstellen. Des Weiteren soll der Einfluss der Pulsfrequenz auf das Blutdruckmessergebnis berücksichtigt werden. Zudem soll eine Verfälschung der Ergebnisse bei hohen und niedrigen Werten, die durch die Festlegung von Standardbedingungen in Form von Amplitudenbegrenzungen, Pulsdefinitionen, etc. entstehen, die extreme Werte unberücksichtigt lassen, vermieden werden.

Diese Aufgabe wird gemäß einem ersten Aspekt der vorliegenden Erfindung durch eine Vorrichtung mit den Merkmalen des Anspruchs 1 gelöst.

Der Prozessor steuert und regelt das elektronische Ablassventil für einzelne Eingangssignale in Echtzeit mittels einer Korrekturfunction, die gewährleistet, dass die Ablassgeschwindigkeit linear mit der Pulsfrequenz und vorzugsweise unter Berücksichtigung der einzelnen Amplituden steigt.

Es wurde überraschender Weise festgestellt, dass die erfindungsgemäßen Vorrichtungen es erlauben, Manschettengrößen weitestgehend unabhängig vom Gliedmaßenumfang und Manschettenvolumen einzusetzen. Zudem ermöglicht die Steuerung und Regelung des elektronischen Ablassventils in Echtzeit die Bestimmung genauer Blutdruckmesswerte für Säugetiere, Vögel oder Reptilien aller Art im Bereich von etwa 5 bis über 300 mm Hg, z.B. bis 450 mm Hg. Vorteilhafter Weise können die erfindungsgemäßen Vorrichtungen Artefakte erkennen. Zudem berücksichtigt die Steuerung des Ablassventils in Echtzeit den Einfluss der Pulsfrequenz, der Pulsamplitude und der Pulsbreite auf das Blutdruckmessergebnis. Auch kann eine Verfälschung der Ergebnisse bei hohen und niedrigen Werten, die durch die bisher gebräuchliche Festlegung von Standardbedingungen in Form von Amplitudenbegrenzungen, Pulsdefinittonen, etc. entstehen konnten, die in diesem Fall extreme Werte unberücksichtigt lassen, vermieden werden.

Die Art der Messmanschette zur Verwendung in der erfindungsgemäßen Vorrichtung kann jede beliebige der handelsüblichen Messmanschetten sein, die eine ausreichende Empfindlichkeit bei der Aufnahme des Signals sowie einen niedrigen Reibungswiderstand aufweist. Zum Beispiel können die Manschetten von Erka (siehe z.B. das deutsche Gebrauchsmuster G 91 06153.9 an R. Kallmeyer) bzw. die in dem deutschen Gebrauchsmuster Nr. 299 08 547 U1 offenbarten Manschetten verwendet wenden. Vorzugsweise ist die Manschette an die Gliedmaßenform und -größe des jeweilig zu untersuchenden Probanden angepasst.

Als Proband kommt jedes Lebewesen mit einem geschlossenen Kreislaufsystem in Frage. Bevorzugte Probanden sind Säugetiere, insbesondere Menschen, aber auch Vögel und Reptilien. Besonders bevorzugte Probanden sind Menschen, Klein- und Haustiere wie Hunde, Katzen, Nager und hasenartige Tiere, aber auch Groß-, Wild- und Nutztiere.

Die Pumpen zur Befüllung der Manschette können für diesen Zweck handelsübliche Pumpen sein, die ein ausreichendes Leistungsverzeichnis wie Pumpgeschwindigkeit, Pumpvolumen, etc. aufzeigen. In einer bevorzugten Ausführungsform wird die Pumpe elektronisch gesteuert und geregelt, mehr bevorzugt über den Prozessor gesteuert und geregelt, Üblicherweise sollte die Pumpe einen Maximaldruck zwischen 300 und 450 mm Hg bereitstellen können.

Die zur Verwendung in der erfindungsgemäßen Vorrichtung geeigneten, elektronischen Ablassventile sollten im vorgegebenen Druckbereich eine zeitlich konstante Druckänderung ermöglichen. Des Weiteren muss das Ablassventil im Wesentlichen verzögerungsfrei geregelt und gesteuert werden können, damit die im Prozessor in Echtzeit bereitgestellten Steuerungs- und Regeldaten ohne oder mit minimaler Verzögerung umgesetzt werden. Geeignete Ablassventile sind unter der Bezeichnung KSV 15C von KOGE, Japan, käuflich verfügbar.

In einer bevorzugten Ausführungsform wird das Signal des Druckaufnehmers durch mindestens einen Signalverstärker zwischen der Messmanschette und dem Analog-/Digitalwandler verstärkt.

Die zur Verwendung in der erfindungsgemäßen Vorrichtung geeigneten, elektronischen Druckaufnehmer sollten im vorgegebenen Druckbereich arbeiten.

In einer bevorzugten Ausführungsform erfolgt eine Vorspannung des Druckaufnehmers durch eine geeignete Strom speisung, die eine geeignete Signalverstärkung und Umrechnung in mV gewährleistet. Geeignete Druckaufnehmer, die eine solche Programmierung ermöglichen, sind unter der Bezeichnung FPN05 von Fujikura, Japan, käuflich verfügbar.

Als Analog-/Digitalwandler kommen handelsübliche Wandler in Frage, die den digitalen Messbereich von mindestens 1024 Bytes abdecken können. Ein Beispiel eines solchen Wandlers ist der Wandler LTC2208 von Linear Technology/USA.

Als Prozessor kommt Jeder handelsübliche Prozessor in Frage, der in der Lage ist, die Daten aus dem Analog-/Digitalwandler in hoher Geschwindigkeit aufzunehmen, und während der Aufnahme der Daten bereits das Ablassventil in Echtzeit zu regeln und zu steuern, so dass eine zeitlich konstante Druckänderung Ober den gesamten gemessenen Druckbereich gewährleistet ist Bevorzugte Prozessoren haben mindestens einen ARM7- oder einen ARM9-Kern (z. B. erhältlich von ATMEL, USA; ANALOG DEVICES, USA; PHILIPS, NL). In bevorzugten Ausführungsformen welsen die Prozessoren ein externes Bus-Interface und/oder RS232/USB-Interface und/oder einen mindestens 10 Bit-Wandler auf.

Unter Steuern und Regeln des elektronischen Ablassventils im Sinne der Erfindung versteht der Fachmann die Einstellung der Ventilparameter wie z.B. Öffnungsdauer, Öffnungsgröße, etc. zur Gewährleistung eines zeitlich konstanten, d.h. linearen, Druckabfalls im gesamten gemessenen Bereich.

Unabdingbar für eine erfindungsgemäße lineare Ventilsteuerung und Regelung ist die Verarbeitung einzelner Eingangssignale und Weiterleitung der Steuerungs- und Regelsignale für das Ventil in Echtzeit. Baubedingt ist je nach Komponentenwahl eine Verzögerung unvermeidbar. Diese sollte jedoch minimiert werden. Der Begriff Echtzeit im Sinne der Erfindung bedeutet, dass die lineare Steuerung des Ablassventils zu jeder Zeit gewährleistet ist.

In einer bevorzugten Ausführungsform wird jedes einzelne Eingangsignal zuerst verifiziert und zur Steuerung und Regelung des Ablassventils herangezogen. Unter Verifizieren ist zu verstehen, dass der Prozessor die einzelnen Eingangssignale durch Vergleich mit den vorangegangenen Daten und/oder bekannten Kenndaten von Artefakten abgleicht, um das Eingangsignal als relevante Blutdruckimpulsinformation zu bestätigen und zu berücksichtigen oder als Artefakt zu verwerfen. Insbesondere bei hohen Pulsraten und Blutdrücken ermöglicht die Verarbeitung aller Eingangssignale eine effiziente und äußerst schnelle sowie genaue Durchführung der Blutdruckmessung. Dieses ist vor allem bei Tieren, aber auch bei Kindern, die üblicher Weise nicht lange stillhalten, von Vorteil. Zum Beispiel kann eine erfindungsgemäße Messung bei einem Kaninchen mit einem Puls von 240 vier mal schneller als bei einem Menschen mit einem Puls von durchschnittlich 60 Pulsschlägen pro Minute durchgeführt werden. Zum Beispiel kann bei einem Kaninchen mit einem Puls von 240 die DruckaMassgeschwindigkeit gegenüber der Messung beim Menschen deutlich vergrößert werden und dennoch eine ausreichende Anzahl von Messpunkten erhalten werden. Eine ausreichende Anzahl von Eingangsignalen wird beim Menschen mit 3 mm Hg pro Sekunde Ablassgeschwindigkeit und 60 Pulsschlägen pro Minute erhalten. Bei 27 mm Hg Druckabfall pro Sekunde und einem Puls von 240 wäre eine Blutdruckmessung beim Kaninchen somit nach 9 Sekunden beendet.

Einzelne Eingangssignale im Sinne der Erfindung bedeutet, dass jedes einzelne, oder auch nur bestimmte, z.B. jedes 2., 3., 4. oder 15. Signal verarbeitet wird. Letztendlich bestimmt die Notwendigkeit der Echlzeitsteuerung und -regelung die Grenzen der Abstände zwischen den Einzelmessungen, d.h. der Abtastrate.

Ein zeitlich konstanter Druckabfall im Sinne der vorliegenden Erfindung liegt vorzugsweise im Bereich von 1 bis 42, mehr bevorzugt bei 6 bis 32, am meisten bevorzugt bei 9 bis 27 mm Hg pro Sekunde.

Der gemessene Druckbereich des Blutdruckmeßgeräts liegt zwischen 450 und 0 mm Hg, vorzugsweise zwischen 300 und 5 mm Hg.

In einer weiteren bevorzugten Ausführungsform steuert und regelt der Prozessor das elektronische Ablassventil für einzelne Eingangssignale in Echtzeit mittels einer Korrekturfunktion, die unterschiedliche Manscheiterrvolumina berücksichtigt, wobei vorzugsweise das Manschettenvolumen über die Zeit zum Befüllen der Messmanschette bestimmt wird.

In einer am meisten bevorzugten Ausführungsform verwendet der Prozessor die folgende Korrekturfunktion:

$$f(x) = K \cdot f(t) \cdot f(p) \cdot (a1 \cdot (x+o1)^3 + a2 \cdot (x+o2)^2 + a3 \cdot (x+o3) + a4/(x+o4) + o5)$$

wobei

f(x) die Ablassfunktion,
K der Umrechnungsfaktor in % Stromimpuls / mm Hg,
f(t) die Funktion der Aufpumpzeit,
t die Aufpumpzeit in ms,
f(p) die Funktion der Pulsfrequenz,
p der Puls in Schlägen pro Minute, und
a1 bis a4 sowie o1 bis o5 für jeden Manschettentyp einzeln zu ermittelnde Korrekttjrkonstanten sind,

wobei vorzugsweise

$$f(t) = 5 + 80 \text{ ms/t};$$

und/oder

$$f(p) = 100 + 60/(\min {}^* p) \text{ ist.}$$

ist.

Ein weiterer Aspekt der vorliegenden Erfindung ist ein Verfahren zur Blutdruckmessung gemäß den Merkmalen des Anspruchs 11.

Das elektronische Ablassventil wird durch den Prozessor für einzelne Eingangssignale in Echtzeit mittels einer Korrekturfunction derart gesteuert und geregelt, dass gewährleistet ist, dass die Ablassgeschwindigkeit linear mit der Pulsfrequenz steigt. Eine solche Ablassfunktion des Ventils gewährleistet auch, dass ein Blutstau verhindert wird.

Die für das erfindungsgemäße Verfahren geeigneten Bauteile bzw. geeignete Vorrichtungen werden oben allgemein sowie als spezielle Ausführungsformen beschrieben.

Der Begriff "Proband" und bevorzugte Probanden zur Durchführung des erfindungsgemäßen Verfahrens wurden bereits oben beschrieben.

Des Weiteren ist es bevorzugt, bei dem Verfahren die Pumpe zum Befüllen der Messmanschette elektronisch zu steuern und zu regeln, vorzugsweise über den Prozessor.

Zur Durchführung des Verfahrens stellt die Pumpe vorzugsweise einen Maximaldruck zwischen 300 mm Hg und 450 mm Hg bereit.

In einer bevorzugten Ausführungsform werden die Messsignale zusätzlich durch mindestens einen Signalversfärker zwischen der Messmanschette und dem Analog-/Digitalwandler verstärkt.

Vorzugsweise weist der Prozessor zur Durchführung des Verfahrens einen ARM7-Kem oder einen ARM9-Kern sowie besonders bevorzugt ein externes Bus-interface und/oder ein RS232/USB-Interface und/oder einen 10Bit-Wandler auf.

In einer bevorzugten Ausführungsform stellt das Verfahren der Erfindung eine Ablassgeschwindigkeit des elektronischen Ablassventils mit einem zeitlich konstanten Druckabfall von 1 bis 42, mehr bevorzugt 6 bis 32, am meisten bevorzugt 9 bis 27 mm Hg pro Sekunde bereit.

Weiterhin bevorzugt sind erfindungsgemäße Verfahren, bei denen das elektronische Ablassventil durch den Prozessor für einzelne Eingangssignale in Echtzeit mittels einer Korrekturfunktion derart gesteuert und geregelt wird, dass unterschiedliche Manschettenvolumina berücksichtigt werden, wobei vorzugsweise das Manschettenvolumen unter anderem über die Zeit zum Befüllen der Messmanschette bestimmt wird. So können die Einflüsse verschiedener Manschettenvolumina, welche durch unterschiedliche Bauformen der Manschetten sowie durch unterschiedlich festes Anlegen entstehen, und welche zu variierenden Messzeiten führen würden, kompensiert werden.

Ganz besonders bevorzugt sind erfindungsgemäße Verfahren, die die oben für die erfindungsgemäße Vorrichtung genannte Korrekturfunktion einsetzen.

Das erfindungsgemäße Verfahren und die erfindungsgemäße Vorrichtung gewährleisten eine lineare Arbeitsweise der Blutdruckmessung, so dass das Luftvolumen der Manschette eines Blutdruckmessgerätes mit konstanter Druckminderung von maximal 450 bis 300 bis minimal 0 mm Hg und unter Berücksichtigung des jeweils einzelnen Amplitudendrucks entweicht. Durch diese konstante Druckminderung über den gesamten Druckbereich werden wesentlich genauere Messwerte für den Blutdruck erhalten als dies mit herkömmlichen Blutdruckmessgeräten oder -verfahren möglich ist.

Das erfindungsgemäße Verfahren und die erfindungsgemäße Vorrichtung erlauben es weiterhin, die Druckablassgeschwindigkeit Im Vergleich mit herkömmlichen Blutdruckmessgeräten oder -verfahren zu erhöhen, wobei dennoch eine ausreichende Anzahl von Messsignalen erhalten wird, um eine exakte Bestimmung der Blutdruckwerte zu gewährleisten. Dadurch kann mit der erfindungsgemäßen Vorrichtung und mit dem erfindungsgemäßen Verfahren eine deutliche schnellere Blutdruckmessung bewerkstelligt werden, als dies mit herkömmlichen Blutdruckmessegeräten oder - verfahren möglich ist.

Im Folgenden wird die Erfindung beispielhaft anhand einer speziellen Ausführungsform illustriert, die in ihren Merkmalen nicht als einschränkend anzusehen ist.

**Figuren**

Fig. 1    zeigt eine typische D-Kurve.

Fig. 2    zeigt eine Korrekturkurve C

Fig. 3    zeigt eine korrigierte Kurve K, d.h. die D-Kurve wurde durch Einberechnung der Korrekturkurve C auf eine virtuelle Nulllinie gebracht.

Fig. 4    zeigt die Bestimmung der Pulse

Fig. 5    zeigt die obere (a) und untere Hüllkurve (b) sowie die Ermittlung von Systole, Diastole und MAP mittels dieser beiden Hüllkurven.

Beispiel 1

Auswertung und Kurve zur Analyse spezifischer Blutdruckmesswerte

Die Messmanschette liefert Daten, bestehend aus dem Druck In mm Quecksiibersäuie (mm Hg) und aus Impulsdaten, die durch einen nachgeschalteten Differenzverstärker verstärkt werden. Die aus dem Differenzverstärker erhaltenen Daten werden vereinfacht auch als D-Kanal bezeichnet. Die gemessenen Werte des D-Kanals ergeben sich aus der Ermittlung der Druckunterschiede der durch das System empfangenen Impulse. Eine nicht exakt lineare Ablassfunktion und eine hohe Verstärkung des D-Kanals führen zu einer Verzerrung der Messdaten.

Daher ist für die Visualisierung, die automatische Analyse und die Bestimmung der relevanten Ergebnisse, wie Systole, Diastole, MAP (mittlerer arterieller Blutdruck) und Puls, eine Aufbereitung der Daten notwendig. Diese Aufbereitung geschieht wie folgt:

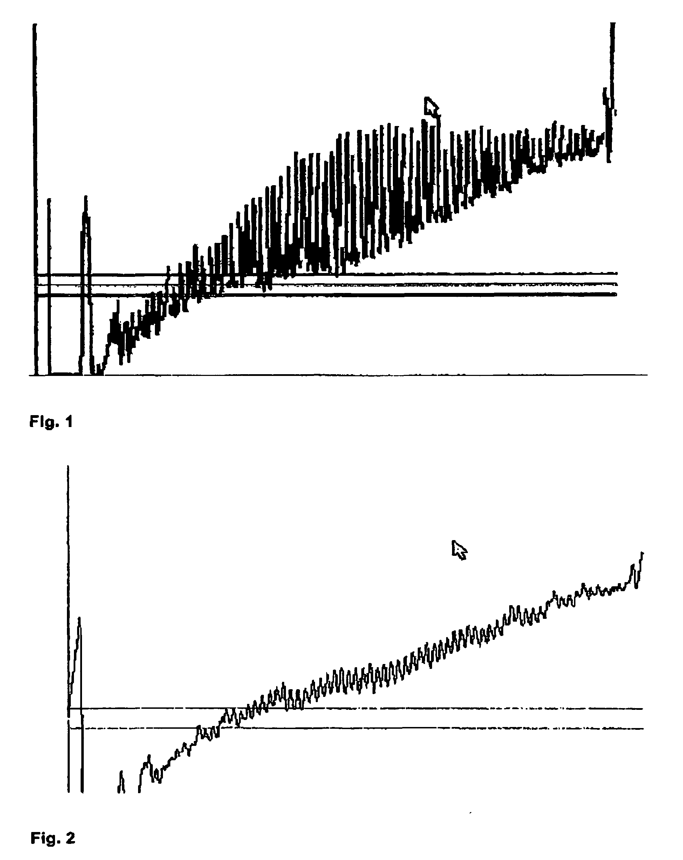

Der aufgezeichnete D-Kanal der Druckmessung liefert eine ansteigende Impulskurve, die auch als D-Kurve bezeichnet wird (siehe Figur 1). Durch Einberechnung einer Korrekturkurve C (siehe Figur 2) wird diese Impulskurve auf eine virtuelle Nulllinie gebracht.

Die Korrekturkurve ergibt sich für jeden Wert der D-Kurve aus der Ableitung:

wenn $(D_{ist} + C_{n-1}) > 2$ dann $C_n = C_{n-1} + 1$

wenn $(D_{ist} + C_{n-1}) < 2$ dann $C_n = C_{n-1} - 1$

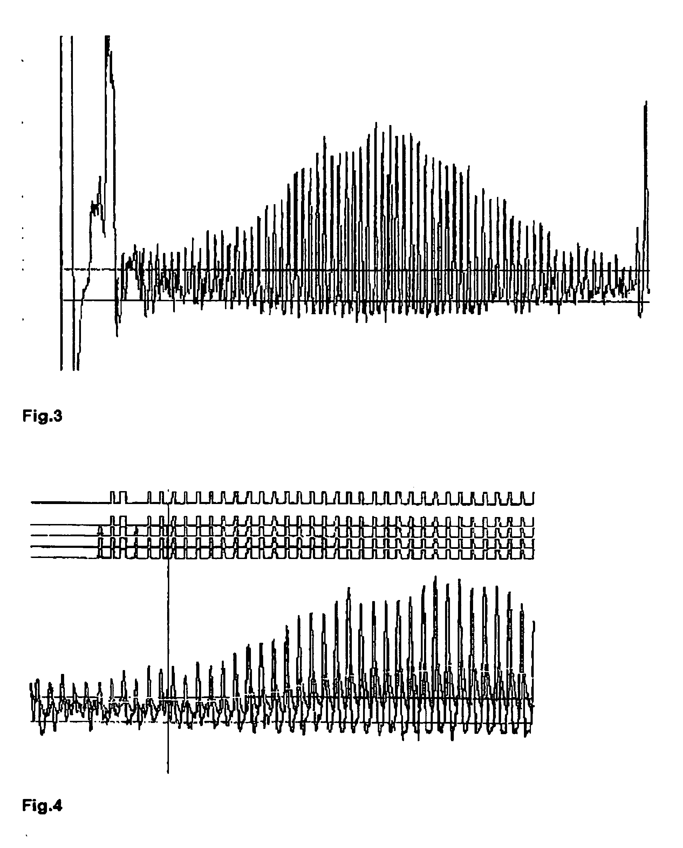

Diese korrigierte Kurve K (siehe Figur 3), d.h. die durch Einberechnung der Korrekturkurve C auf eine virtuelle Nulllinie gebrachte D-Kurve, wird für die weitere Auswertung verwendet.

Es wird ein Schwellenwert gesetzt, der zur Bestimmung der Pulse (siehe Figur 4) dient. Damit eine Pulszacke akzeptiert wird, muss sie 4 Kriterien erfüllen:

a) der Puls muss eine Mindestbreite aufweisen,
b) der Puls darf eine Maximalbreite nicht überschreiten,
c) der Puls muss eine Mindesthöhe aufweisen,
d) der Puls darf eine Maximalhöhe nicht überschreiten.

Nach Durchführung dieses Schrittes Ist die Position jeder einzelnen Pulszacke bekannt.

Die so ermittelten Punkte dienen als Kontrollkurve einer Bezierkurve (Normierungskurve) höherer Ordnung.

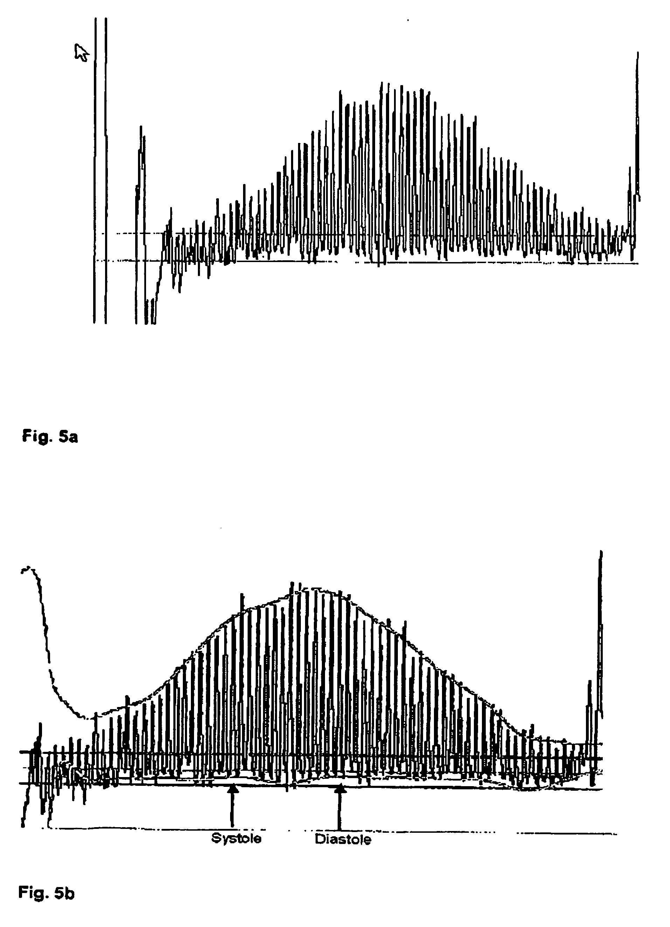

Um die normierte Kurve wird eine obere und eine untere Hüllkurve gelegt. Die Hüllkurven werden nach dem gleichen Verfahren wie die Normierungskurve aus den Minima und den Maxima der normierten Kurve berechnet (siehe Figur 5).

Schließlich werden die obere Hüllkurve und die untere Hüllkurve analysiert, um Systole, Diastole und MAP zu ermitteln.

Die Systole ergibt sich aus dem ersten lokalen Maximum der unteren Hüllkurve, das sich _links_ vom Maximum der oberen Hüllkurve befindet.

Die Diastole ergibt sich aus dem ersten lokalen Maximum der unteren Hüllkurve, das sich _rechts_ vom Maximum der oberen Hüllkurve befindet.

Der mittlere arterielle Blutdruck MAP errechnet sich dadurch, dass die Flächen der drei Pulszacken um das Maximum der oberen Hüllkurve herum gemittelt werden.

**Patentansprüche**

**1.**  Eine Vorrichtung zur Blutdruckmessung, umfassend

a) eine Messmanschette,
b) einen Druckaufnehmer,
c) eine Pumpe zum Befüllen der Messmanschette,
d) ein elektronisches Ablassventil,

e) einen Analog-/Digitalwandler, und

f) einen Prozessor zur Steuerung und Regelung des elektronischen Ablassventils,

wobei die aufgenommenen Daten von der Messmanschette über den Druckaufnehmer und den Analog-/Digitalwandler an den Prozessor weiterleitbar sind, wobei der Prozessor derart ausgebildet ist, dass dieser das elektronische Ablassventil für einzelne Eingangssignale in Echtzeit derart steuert und regelt, dass ein Druckabfall über den gesamten gemessenen Druckbereich zeitlich konstant ist,
**dadurch gekennzeichnet,**
**dass** der Prozessor derart ausgebildet ist, dass dieser das elektronische Ablassventil für einzelne Eingangssignale in Echtzeit mittels einer Korrekturfunktion derart steuert und regelt , dass die Ablassgeschwindigkeit linear mit einer Pulsfrequenz steigt.

2.  Die Vorrichtung gemäß Anspruch 1, wobei zusätzlich die Pumpe elektronisch Steuerbar und regelbar ist, vorzugsweise über den Prozessor.

3.  Die Vorrichtung gemäß Anspruch 1 oder 2, wobei die Pumpe auf einen Maximaldruck zwischen 300 mm Hg und 450 mm Hg einstellbar ist.

4.  Die Vorrichtung gemäß einem der Ansprüche 1 bis 3, wobei die Vorrichtung zusätzlich mindestens einen Signalverstärker zwischen der Messmanschette und dem Analog-/Digitalwandler aufweist.

5.  Die Vorrichtung gemäß einem der Ansprüche 1 bis 4, wobei der Prozessor einen ARM7-Kern oder einen ARM9-Kern aufweist.

6.  Die Vorrichtung gemäß einem der Ansprüche 1 bis 5, wobei der Prozessor ein externes Bus-Interface und/oder ein RS232/USB-Interface und/oder einen mindestens 10Bit-Wandler aufweist.

7.  Die Vorrichtung gemäß einem der Ansprüche 1 bis 6, wobei der zeitlich konstante Druckabfall zwischen 1 bis 42, mehr bevorzugt 6 bis 32, am meisten bevorzugt 9 bis 27 mm Hg pro Sekunde liegt.

8.  Die Vorrichtung gemäß einem der Ansprüche 1 bis 7, wobei der zeitlich konstante Druckabfall unter Berücksichtigung der Amplituden der einzelnen Eingangssignale veränderbar ist.

9.  Die Vorrichtung gemäß Anspruch 1, wobei der Prozessor derart ausgebildet ist das elektronische Ablassventil für einzelne Eingangssignale in Echtzeit mittels der Korrekturfunktion zu steuern und regeln und, dass unterschiedliche Manschettenvolumina berücksichtigt werden, wobei vorzugsweise das Manschettenvolumen über die Zeit zum Befüllen der Messmanschette bestimmt wird.

10. Die Vorrichtung gemäß einem der Ansprüche 1 oder 9, wobei der Prozessor ausgebildet ist die Korrekturfunktion:

$$f(x) = K * f(t) * f(p) * (a1*(x+o1)^3 + a2*(x+o2)^2 + a3*(x+o3) + a4/(x+o4) + o5)$$

einzusetzen, wobei

f(x) die Ablassfunktion,
K der Umrechnungsfaktor in % Stromimpuls / mm Hg,
f(t) die Funktion der Aufpumpzeit,
t die Aufpumpzeit in ms,
f(p) die Funktion der Pulsfrequenz,
p der Puls in Schlägen pro Minute, und
a1 bis a4 sowie o1 bis o5 für jeden Manschettentyp einzeln zu ermittelnde Korrekturkonstanten sind,

wobei vorzugsweise

f(t) = 5 + 80 ms/t; und/oder
f(p) = 100 + 60/(min * p) ist.

11. Ein Verfahren zur Blutdruckmessung, umfassend

a) das Anlegen einer geeigneten Blutdruckmessmanschette als Druckaufnehmer um eine Gliedmaße des Probanden mit einem geschlossenen Kreislaufsystem, wie Säuger, Vogel oder Reptil sowie das ausreichende Befüllen mit einer Pumpe;
b) das Ablassen des Drucks der Messmanschette mittels eines elektronisch gesteuerten Ventils während der Messung des Blutdrucks;
c) die Umwandlung der analogen Messsignale der Messmanschette in einem Analog-/Digitalwandler; und
d) die Verarbeitung der digitalen Messsignale durch den Prozessor, wobei der Prozessor das elektronische Ablassventil für einzelne Eingangssignale in Echtzeit derart steuert und regelt, dass ein Druckabfall über den gesamten gemessenen Druckbereich zeitlich konstant ist,

**dadurch gekennzeichnet,**
**dass** der Prozessor das elektronische Ablassventil für einzelne Eingangssignale in Echtzeit mittels einer Korrekturfunktion derart steuert und regelt, dass die Ablassgeschwindigkeit linear mit der Pulsfrequenz steigt.

12. Das Verfahren gemäß Anspruch 11, wobei die Pumpe elektronisch gesteuert und geregelt wird, vorzugsweise über den Prozessor.

13. Das Verfahren gemäß einem der Ansprüche 11 oder 12, wobei die Pumpe einen Maximaldruck zwischen 300 mm Hg und 450 mm Hg bereitstellt.

14. Das Verfahren gemäß Anspruch 11, wobei die Messsignale zusätzlich durch mindestens einen Signalverstärker zwischen der Messmanschette und dem Analog-/Digitalwandler verstärkt werden.

15. Das Verfahren gemäß einem der Ansprüche 11 bis 13, wobei das elektronische Ablassventil einen zeitlich konstanten Druckabfall von 1 bis 42, mehr bevorzugt 6 bis 32, am meisten bevorzugt 9 bis 27 mm Hg pro Sekunde bereitstellt.

16. Das Verfahren gemäß einem der Ansprüche 11 bis 15, wobei das elektronische Ablassventil durch den Prozessor für einzelne Eingangssignale in Echtzeit mittels der Korrekturfunktion derart gesteuert und geregelt wird, dass der zeitlich konstante Druckabfall unter Berücksichtigung der Amplituden der einzelnen Eingangssignale verändert wird.

17. Das Verfahren gemäß einem der Ansprüche 10 bis 14, wobei das elektronische Ablassventil durch den Prozessor für einzelne Eingangssignale in Echtzeit mittels der Korrekturfunktion derart gesteuert und geregelt wird, dass unterschiedliche Manschettenvolumina berücksichtig werden wobei vorzugsweise das Manschettenvolumen über die Zeit zum Befüllen der Messmanschette bestimmt wird.

18. Das Verfahren gemäß einem der Ansprüche 10 bis 17, wobei die Korrekturfunktion:

$$f(x) = K * f(t) * f(p) * (a1*(x+o1)^3 + a2*(x+o2)^2 + a3*(x+o3) + a4/(x+o4) + o5)$$

ist, wobei

f(x) die Ablassfunktion,
K der Umrechnungsfaktor in % Stromimpuls / mm Hg,
f(t) die Funktion der Aufpumpzeit,
t die Aufpumpzeit in ms,
f(p) die Funktion der Pulsfrequenz,
p der Puls in Schlägen pro Minute, und

a1 bis a4 sowie o1 bis o5 für jeden Manschettentyp einzeln zu ermittelnde Korrekturkonstanten sind,
wobei vorzugsweise
f(t) = 5 + 80 ms/t; und/oder
f(p) = 100 + 60/(min * p) ist.

**Claims**

1. A device for measuring blood pressure, comprising

   a) a measuring cuff,
   b) a pressure sensor,
   c) a pump for filling the measuring cuff,
   d) an electronic outlet valve,
   e) an analogue-to-digital converter, and
   f) a processor for open- and closed-loop control of the electronic outlet valve,

   the recorded data from the measuring cuff being relayable to the processor via the pressure sensor and the analogue-to-digital converter, the processor being configured in such a way that it open- and closed-loop controls the electronic outlet valve for individual input signals in real time in such a way that a pressure drop is constant over time over the entire measured pressure range,
   **characterised in that**
   the processor is configured such that it open- and closed-loop controls the electronic outlet valve for individual input signals in real time by means of a correction function in such a way that the outlet rate increases in linear manner with a pulse frequency.

2. The device according to claim 1, wherein the pump is additionally electronically open- and closed-loop controllable, preferably via the processor.

3. The device according to claim 1 or claim 2, wherein the pump is adjustable to a maximum pressure of between 300 mm Hg and 450 mm Hg.

4. The device according to any one of claims 1 to 3, wherein the device additionally comprises at least one signal amplifier between the measuring cuff and the analogue-to-digital converter.

5. The device according to any one of claims 1 to 4, wherein the processor comprises an ARM7 core or an ARM9 core.

6. The device according to any one of claims 1 to 5, wherein the processor comprises an external bus interface and/or an RS232/USB interface and/or an at least 10 bit converter.

7. The device according to any one of claims 1 to 6, wherein the constant pressure drop over time is between 1 to 42, more preferably 6 to 32, most preferably 9 to 27 mm Hg per second.

8. The device according to any one of claims 1 to 7, wherein the constant pressure drop over time is variable taking account of the amplitudes of the individual input signals.

9. The device according to claim 1, wherein the processor is configured in such a way as to open- and closed-loop control the electronic outlet valve for individual input signals in real time by means of the correction function, and wherein different cuff volumes are taken into account, wherein preferably the cuff volume is determined over the time for filling the measuring cuff.

10. The device according to any one of claims 1 or 9, wherein the processor is configured to use the correction function:

$$f(x) = K * f(t) * f(p) * (a1*(x+o1)^3 + a2*(x+o2)^2 + a3*(x+o3) + a4/(x+o4) + o5),$$

   wherein

   $f(x)$ is the outlet function,
   K is the conversion factor in % current pulse / mm Hg,
   $f(t)$ is the inflation time function,
   t is the inflation time in ms,

f(p) is the pulse frequency function,
p is the pulse in beats per minute, and

a1 to a4 and o1 to o5 are correction constants to be individually determined for each cuff type, wherein preferably
f(t) = 5 + 80 ms/t; and/or
f(p) = 100 + 60/(min * p).

**11.** A method for measuring blood pressure, comprising

a) application of a suitable blood pressure measuring cuff as a pressure sensor around a limb of the test subject with a closed circulatory system, such as mammals, birds or reptiles and sufficient filling with a pump;
b) letting the pressure out of the measuring cuff by means of an electronically controlled valve during blood pressure measurement;
c) conversion of the analogue measurement signals of the measuring cuff in an analogue-to-digital converter; and
d) processing of the digital measurement signals by the processor, the processor open- and closed-loop controlling the electronic outlet valve for individual input signals in real time in such a way that a pressure drop over the entire measured pressure range is constant over time,

**characterised in that**
the processor open- and closed-loop controls the electronic outlet valve for individual input signals in real time by means of a correction function in such a way that the outlet rate rises in linear manner with the pulse frequency.

**12.** The method according to claim 11, wherein the pump is electronically open- and closed-loop controlled, preferably via the processor.

**13.** The method according to one of claims 11 or 12, wherein the pump provides a maximum pressure of between 300 mm Hg and 450 mm Hg.

**14.** The method according to claim 11, wherein the measurement signals are additionally amplified by at least one signal amplifier between the measuring cuff and the analogue-to-digital converter.

**15.** The method according to one of claims 11 to 13, wherein the electronic outlet valve provides a constant pressure drop over time of 1 to 42, more preferably 6 to 32, most preferably 9 to 27 mm Hg per second.

**16.** The method according to one of claims 11 to 15, wherein the electronic outlet valve is open- and closed-loop controlled by the processor for individual input signals in real time by means of the correction function in such a way that the constant pressure drop over time is modified taking account of the amplitudes of the individual input signals.

**17.** The method according to one of claims 10 to 14, wherein the electronic outlet valve is open- and closed-loop controlled by the processor for individual input signals in real time by means of the correction function in such a way that different cuff volumes are taken into account, wherein the cuff volume is preferably determined over the time for filling the measuring cuff.

**18.** The method according to one of claims 10 to 17, wherein the correction function is:

$$f(x) = K * f(t) * f(p) * (a1*(x+o1)^3 + a2*(x+o2)^2 + a3*(x+o3) + a4/(x+o4) + o5),$$

wherein

f(x) is the outlet function,
K is the conversion factor in % current pulse / mm Hg,
f(t) is the inflation time function,
t is the inflation time in ms,
f(p) is the pulse frequency function,

p is the pulse in beats per minute, and

a1 to a4 and o1 to o5 are correction constants to be individually determined for each cuff type, wherein preferably
f(t) = 5 + 80 ms/t; and/or
f(p) = 100 + 60/(min*p).

**Revendications**

1. Un appareil de mesure de la pression artérielle, comprenant

a) une manchette de mesure,
b) un capteur de pression
c) une pompe permettant le remplissage de la manchette de mesure,
d) une vanne de décompression électronique,
e) un convertisseur analogique/numérique, et
f) un processeur de commande et de réglage de la vanne de décompression électronique,

sachant que les données enregistrées par la manchette de mesure sont transmissibles au processeur par le biais du capteur de pression et du convertisseur analogique/numérique, sachant que le processeur est conçu de sorte qu'il commande et module en temps réel la vanne de décompression électronique pour chaque signal d'entrée, de sorte que la chute de pression est constante dans le temps sur toute la plage de pression mesurée, **caractérisé en ce que** le processeur est conçu de sorte qu'il commande et module en temps réel la vanne de décompression électronique pour chaque signal d'entrée à l'aide d'une fonction de correction, de sorte que la vitesse de décompression augmente linéairement avec la fréquence du pouls.

2. L'appareil selon la revendication 1, sachant qu'en plus la pompe peut être commandée et modulée par voie électronique, de préférence à l'aide du processeur.

3. L'appareil selon la revendication 1 ou 2, sachant que la pompe peut être réglée à une pression maximale entre 300 mm Hg et 450 mm Hg.

4. L'appareil selon l'une des revendications 1 à 3, sachant que l'appareil comprend en plus au moins un amplificateur de signaux entre la manchette de mesure et le convertisseur analogique/numérique.

5. L'appareil selon l'une des revendications 1 à 4, sachant que le processeur comprend un noyau ARM7 ou un noyau ARM9.

6. L'appareil selon l'une des revendications 1 à 5, sachant que le processeur comprend une interface bus externe et/ou une interface RS232/USB et/ou un convertisseur avec au moins 10 bits.

7. L'appareil selon l'une des revendications 1 à 6, sachant que la chute de pression constante dans le temps se situe entre 1 et 42, mieux encore entre 6 et 32, et encore plus avantageusement entre 9 et 27 mm Hg par seconde.

8. L'appareil selon l'une des revendications 1 à 7, sachant que la chute de pression constante dans le temps peut être modifiée en fonction des amplitudes de chaque signal d'entrée.

9. L'appareil selon la revendication 1, sachant que le processeur est conçu de sorte qu'il commande et module en temps réel la vanne de décompression électronique pour chaque signal d'entrée à l'aide de la fonction de correction et que différents volumes de manchette sont pris en compte, sachant que de préférence le volume de manchette est déterminé en fonction du temps de remplissage de la manchette de mesure.

10. L'appareil selon l'une des revendications 1 ou 9, sachant que le processeur est conçu pour appliquer la fonction de correction :

$$f(x) = K * f(t) * f(p) * (a1*(x+o)^3 + a2*(x+o2)^2 + a3*(x+o3) + a4/(x+o4) + o5),$$

dans laquelle

f(x) la fonction de décompression,
K le facteur de conversion en % impulsion de courant / mm Hg,
f(t) la fonction du temps de gonflage,
t le temps de gonflage en ms,
f(p) la fonction de fréquence du pouls,
p le pouls en battements par minute, et

a1 à a4 ainsi que o1 à o5 sont des constantes de correction à déterminer individuellement pour chaque type de manchette,
sachant que de préférence
(t) = 5 + 80 ms/t ; et/ou
(p) = 100 + 60/(min * p).

11. Un procédé de mesure de la pression artérielle, comprenant

a) la pose d'une manchette de mesure de la pression sanguine appropriée et faisant office de capteur de pression autour d'un membre d'un sujet expérimental ayant un système circulatoire fermé, tel un mammifère, oiseau ou reptile ainsi qu'un remplissage suffisant à l'aide d'une pompe;
b) la décompression de la pression de la manchette de mesure à l'aide d'une vanne à commande électronique pendant la mesure de la pression sanguine ;
c) la conversion des signaux de mesure analogiques de la manchette de mesure dans un convertisseur analogique/numérique ; et
d) le traitement des signaux de mesure numériques par le processeur, sachant que le processeur commande et module en temps réel la vanne de décompression électronique pour chaque signal d'entrée, de sorte qu'une chute de pression reste constante dans le temps sur toute la plage de pression mesurée,

**caractérisé en ce**
**que** le processeur commande et module en temps réel la vanne de décompression électronique pour chaque signal d'entrée à l'aide d'une fonction de correction, de sorte que la vitesse de décompression augmente linéairement avec la fréquence du pouls.

12. Le procédé selon la revendication 11, sachant que la pompe peut être commandée et modulée par voie électronique, de préférence à l'aide du processeur.

13. Le procédé selon l'une des revendications 11 ou 12, sachant que la pompe produit une pression maximale entre 300 mm Hg et 450 mm Hg.

14. Le procédé selon la revendication 11, sachant que les signaux de mesure sont amplifiés en plus par au moins un amplificateur de signaux situé entre la manchette de mesure et le convertisseur analogique/numérique.

15. Le procédé selon l'une des revendications 11 à 13, sachant que la vanne de décompression électronique produit une chute de pression constante dans le temps située entre 1 et 42, mieux encore entre 6 et 32, encore plus avantageusement entre 9 et 27 mm Hg par seconde.

16. Le procédé selon l'une des revendications 11 à 15, sachant que la vanne de décompression électronique est commandée et modulée en temps réel par le processeur pour chaque signal d'entrée à l'aide d'une fonction de correction, de sorte que la chute de pression constante dans le temps est modifiée en tenant compte des amplitudes de chaque signal d'entrée.

17. Le procédé selon l'une des revendications 10 à 14, sachant que la vanne de décompression électronique est commandée et modulée en temps réel par le processeur pour chaque signal d'entrée à l'aide de la fonction de correction, de sorte qu'il est possible de prendre en compte différents volumes de manchette, sachant que de préférence le volume de la manchette est déterminé en fonction du temps de remplissage de la manchette de mesure.

18. Le procédé selon l'une des revendications 10 à 17, sachant que la fonction de correction est :

$$f(x) = K * f(t) * f(p) * (a1*(x+o1)^3 + a2*(x+o2)^2 + a3*(x+o3) + a4/(x+o4) + o5),$$

dans laquelle

f(x) la fonction de décompression,
K le facteur de conversion en % impulsion de courant / mm Hg,
f(t) la fonction du temps de gonflage,
t le temps de gonflage en ms,
f(p) la fonction de la fréquence du pouls,
p le pouls en battements par minute, et

a1 à a4 ainsi que o1 à o5 sont des constantes de correction à déterminer individuellement pour chaque type de manchette,
sachant que de préférence
f(t) = 5 + 80 ms/t ; et/ou
f(p) = 100 + 60/(min * p).

Flg. 1

Fig. 2

**Fig.3**

**Fig.4**

Fig. 5a

Fig. 5b

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 4300966 A
- DE 4230693 A
- US 6068601 B
- EP 0651969 A
- DE 19700115 A
- EP 1057450 A
- DE G9106153
- DE 29908547 U1

### In der Beschreibung aufgeführte Nicht-Patentliteratur

- **R. L. STEPIEN et al.** Comparative diagnostic test characteristics of oscillometric and doppler ultrasonographic methods in the detection of systolic hypertension in dogs. *J. Vet. Intern. Med.,* 2003, vol. 17, 65-72